(19) Europäisches Patentamt
European Patent Office
Office européen des brevets

(11) Veröffentlichungsnummer: **0 111 581**
**A1**

(12) # EUROPÄISCHE PATENTANMELDUNG

(21) Anmeldenummer: 82111767.8

(22) Anmeldetag: 18.12.82

(51) Int. Cl.³: **C 07 C 76/02**
**C 07 C 79/46**

(43) Veröffentlichungstag der Anmeldung:
27.06.84 Patentblatt 84/26

(84) Benannte Vertragsstaaten:
AT BE CH FR GB IT LI

(71) Anmelder: **BAYER AG**
**Konzernverwaltung RP Patentabteilung**
**D-5090 Leverkusen 1 Bayerwerk(DE)**

(72) Erfinder: **Sehnem, Hans Peter**
**Nüller Strasse 90**
**D-5600 Wuppertal 1(DE)**

(54) **Verfahren zur Herstellung von 5-(2,6-Dichlor-4-trifluor-methyl-phenoxy)-2-nitro-alpha-phenoxy-propionsäure-(hydroxyessigsäureethylester)-ester.**

(57) Verfahren zur Herstellung der Verbindung der Formel

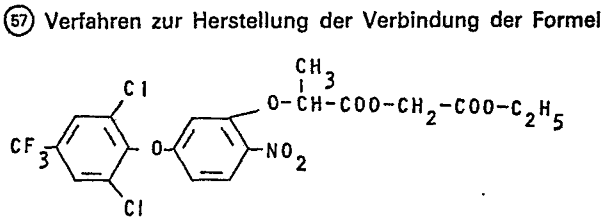

(I)

durch Umsetzung der Verbindung der Formel

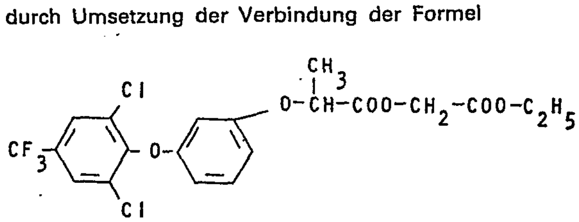

(II)

mit Salpetersäure in Gegenwart von Schwefelsäure sowie in Gegenwart von 1,2-Dichlorethan bei Temperaturen zwischen −20°C und +50°C.

EP 0 111 581 A1

BAYER AKTIENGESELLSCHAFT 5090 Leverkusen, Bayerwerk

Zentralbereich
Patente, Marken und Lizenzen Dü-by-c

IVa

# Verfahren zur Herstellung von 5-(2,6-Dichlor-4-trifluormethyl-phenoxy)-2-nitro-α-phenoxy-propionsäure-(hydroxyessigsäureethylester)-ester

Die Erfindung betrifft ein neues Verfahren zur Herstellung des bekannten 5-(2,6-Dichlor-4-trifluormethyl-phenoxy)-2-nitro-α-phenoxy-propionsäure-(hydroxyessigsäureethylester)-esters, welcher herbizide Eigenschaften besitzt.

Es ist bereits bekannt geworden, daß sich der 5-(2,6-Dichlor-4-trifluormethyl-phenoxy)-2-nitro-α-phenoxy-propionsäure-(hydroxyessigsäureethylester)-ester durch Umsetzung von 5-(2,6-Dichlor-4-trifluormethyl-phenoxy)-2-nitro-α-phenoxy-propionsäurechlorid mit Hydroxyessigsäureethylester in Gegenwart eines Säurebindemittels herstellen läßt (vgl. DE-OS 29 06 087). Der entscheidende Nachteil dieses Verfahrens besteht darin, daß das gewünschte Produkt in einer für praktische Zwecke zu geringen Ausbeute anfällt.

Es wurde nun gefunden, daß man den bekannten 5-(2,6-Dichlor-4-trifluormethyl-phenoxy)-2-nitro- α -phenoxy-propionsäure-(hydroxyessigsäureethylester)-ester der Formel

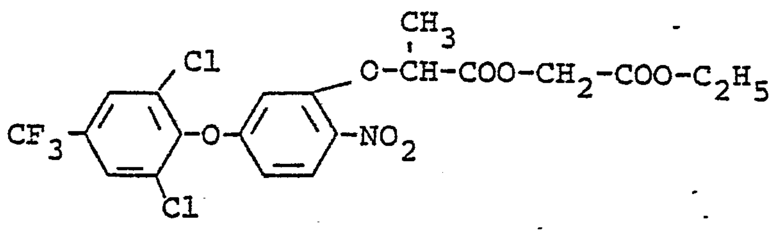

(I)

erhält, wenn man 3-(2,6-Dichlor-4-trifluormethyl-phenoxy)-α -phenoxy-propionsäure-(hydroxyessigsäureethylester)-ester der Formel

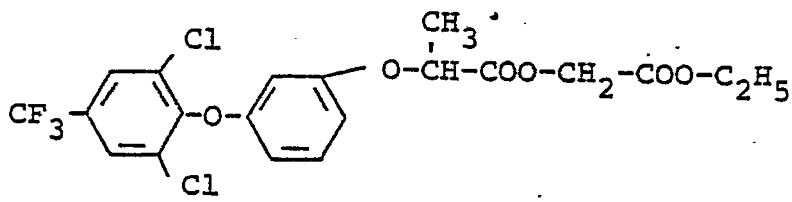

(II)

mit Salpetersäure in Gegenwart von Schwefelsäure sowie in Gegenwart von 1,2-Dichlorethan bei Temperaturen zwischen -20°C und +50°C umsetzt.

Es ist als ausgesprochen überraschend zu bezeichnen, daß sich der 5-(2,6-Dichlor-4-trifluormethyl-phenoxy)-2-nitro- α -phenoxy-propionsäure-(hydroxyessigsäureethylester)-ester der Formel (I) nach dem erfindungsgemäßen Verfahren in sehr guter Reinheit und extrem hoher Ausbeute herstellen läßt, denn bei der analogen

Nitrierung des 3-(2,6-Dichlor-4-trifluormethyl-phenoxy)-α-phenoxy-propionsäure-(hydroxyessigsäuremethylester)-esters wird der entsprechende nitrierte Methylester nur in relativ geringer Ausbeute erhalten. Ein derart unterschiedlicher Reaktionsverlauf bei der Nitrierung homologer Diphenylether-Derivate konnte aufgrund des bekannten Standes der Technik nicht erwartet werden.

Das erfindungsgemäße Verfahren besitzt eine Reihe von Vorteilen. So sind die als Ausgangsprodukte benötigten Substanzen auch in größeren Mengen in einfacher Weise zugänglich und auch im technischen Maßstab problemlos zu handhaben. Ferner ist der zur Durchführung des erfindungsgemäßen Verfahrens erforderliche apparative Aufwand gering und die Aufarbeitung des nach beendeter Umsetzung anfallenden Reaktionsgemisches bereitet keine Schwierigkeiten. Insbesondere jedoch läßt sich der 5-(2,6-Dichlor-4-trifluormethyl-phenoxy)-2-nitro-α-phenoxy-propionsäure-(hydroxyessigsäureethylester)-ester der Formel (I) nach dem erfindungsgemäßen Verfahren in wesentlich höherer Ausbeute herstellen als nach der bisher bekannten Synthesemethode. Das erfindungsgemäße Verfahren stellt somit eine wertvolle Bereicherung der Technik dar.

Der Verlauf des erfindungsgemäßen Verfahrens läßt sich durch das folgende Formelschema veranschaulichen:

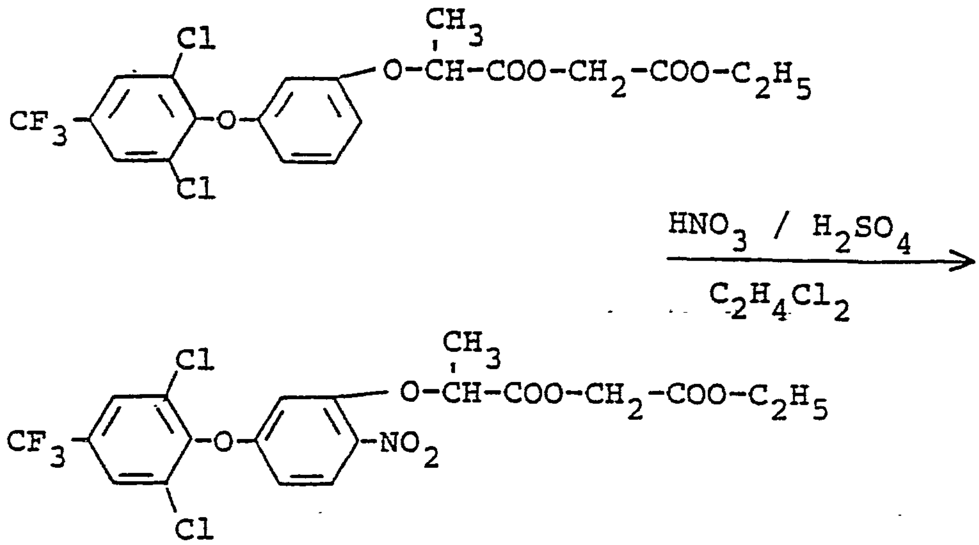


Der bei dem erfindungsgemäßen Verfahren als Ausgangsstoff benötigte 3-(2,6-Dichlor-4-trifluormethyl-phenoxy)-α-phenoxy-propionsäure-(hydroxyessigsäureethylester)-ester der Formel (II) ist bereits bekannt (vgl. DE-OS 28 05 981). Die betreffende Verbindung läßt sich zum Beispiel dadurch herstellen, daß man 3-(2,6-Dichlor-4-trichlormethyl-phenoxy)-phenol der Formel

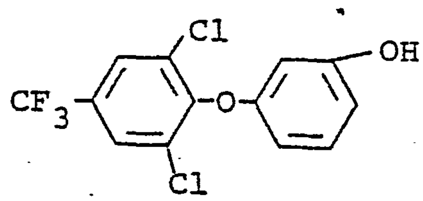

(III)

mit α-Brompropionsäure-(hydroxyessigsäureethylester)-ester der Formel

$$Br-\overset{CH_3}{CH}-COO-CH_2-COOC_2H_5 \qquad (IV)$$

in Gegenwart eines Säurebindemittels, wie zum Beispiel

Kaliumcarbonat, und in Gegenwart eines inerten Verdünnungsmittels, wie zum Beispiel Aceton, bei einer Temperatur zwischen 40 und 70°C umsetzt. Die Aufarbeitung erfolgt nach üblichen Methoden.

Die Nitrierung wird bei dem erfindungsgemäßen Verfahren mit einem Gemisch aus Salpetersäure und Schwefelsäure durchgeführt. Vorzugsweise verwendet man rauchende Salpetersäure und konzentrierte oder zumindest stark konzentrierte Schwefelsäure. Ganz besonders geeignet ist ein Gemisch aus 96 %iger Salpetersäure und 96 %iger Schwefelsäure.

Bei der Umsetzung nach dem erfindungsgemäßen Verfahren dient 1,2-Dichlorethan als Verdünnungsmittel. Es ist jedoch auch möglich, das 1,2-Dichlorethan teilweise oder ganz durch andere chlorierte Kohlenwasserstoffe, wie Tetrachlorkohlenstoff oder Chloroform, zu ersetzen.

Die Reaktionstemperaturen können bei dem erfindungsgemäßen Verfahren innerhalb eines bestimmten Bereiches variiert werden. Vorzugsweise arbeitet man bei Temperaturen zwischen -15°C und +30°C, ganz besonders bevorzugt zwischen -10°C und +10°C.

Das erfindungsgemäße Verfahren wird im allgemeinen unter Normaldruck durchgeführt.

Bei der Durchführung des erfindungsgemäßen Verfahrens setzt man auf 1 Mol 3-(2,6-Dichlor-4-trifluormethyl-phenoxy)-α-phenoxy-propionsäure-(hydroxyessigsäure-ethylester)-ester der Formel (II) 1 bis 10 Mol, vorzugsweise 2,5 bis 7,5 Mol Salpetersäure und 0,5 bis 10 Mol, vorzugsweise 1 bis 5 Mol Schwefelsäure ein. Im einzelnen verfährt man in der Weise, daß man die Verbindung der Formel (II) in 1,2-Dichlorethan löst und in diese Lösung unter Rühren und Kühlung das zur Nitrierung verwendete Gemisch aus Salpetersäure und Schwefelsäure eintropft. Die Isolierung des Reaktionsproduktes erfolgt nach üblichen Methoden. Im allgemeinen geht man so vor, daß man das Reaktionsgemisch mit Wasser versetzt, die wäßrige Phase mehrfach mit einem organischen Lösungsmittel, wie zum Beispiel 1,2-Dichlorethan, extrahiert. Anschließend wäscht man die vereinigten organischen Phasen und zieht dann das Lösungsmittel unter vermindertem Druck ab. Den verbleibenden öligen Rückstand überführt man durch Behandlung mit geeigneten organischen Solventien, wie zum Beispiel Isopropanol oder Cyclohexan, in den festen Zustand und kristallisiert gegebenenfalls um aus einem Alkohol, wie zum Beispiel Isopropanol, aus Cyclohexan oder auch aus Wasser.

Das erfindungsgemäße Verfahren kann sowohl kontinuierlich als auch diskontinuierlich durchgeführt werden.

Der nach dem erfindungsgemäßen Verfahren herstellbare 5-(2,6-Dichlor-4-trifluormethyl-phenoxy)-2-nitro-α-phenoxy-propionsäure-(hydroxyessigsäureethylester)-ester

besitzt herbizide Eigenschaften (vgl. DE-OS 29 06 087). Er eignet sich insbesondere zur selektiven Unkrautbekämpfung.

Das erfindungsgemäße Verfahren und dessen Überlegenheit gegenüber dem bisherigen Verfahren zur Herstellung der Verbindung der Formel (I) sowie der überraschende Verlauf des erfindungsgemäßen Verfahrens im Vergleich zur analogen Nitrierung des 3-(2,6-Dichlor-4-trifluormethyl-phenoxy)-α-phenoxy-propionsäure-(hydroxyessigsäure-methylester)-esters werden durch die nachfolgenden Beispiele veranschaulicht.

Herstellungsbeispiele

Beispiel 1

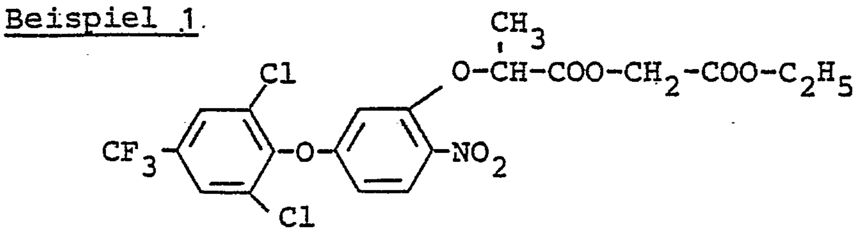

Zu einer Lösung von 120,3 g (0,25 Mol) 3-(2,6-Dichlor-4-trifluormethyl-phenoxy)-α-phenoxy-propionsäure-(hydroxyessigsäureethylester)-ester in 1000 ml 1,2-Dichlorethan wird bei einer Temperatur zwischen -10°C und -5°C unter Rühren eine Mischung aus 64 g (0,625 Mol) 96 %iger Schwefelsäure und 82 g (1,25 Mol) 96 %iger Salpetersäure getropft. Nach beendeter Zugabe wird weitere 60 Minuten bei -10°C bis -5°C gerührt. Anschließend versetzt man das Reaktionsgemisch unter Kühlung mit 350 ml Wasser und trennt die Phasen. Die wäßrige Phase wird mit 200 ml 1,2-Dichlorethan extrahiert. Die vereinigten organischen Phasen werden mit 200 ml Wasser gewaschen und dann unter vermindertem Druck durch Abziehen des Lösungsmittels eingeengt. Es verbleibt ein rotbraunes, zähflüssiges Öl, das nach Zugabe von Isopropanol kristallisiert. Der Feststoff wird abfiltriert und getrocknet. Man erhält auf diese Weise 120,2 g (91,4 % der Theorie) an 5-(2,6-Dichlor-4-trifluormethyl-phenoxy)-2-nitro-α-phenoxy-propionsäure-(hydroxyessigsäureethylester)-ester in Form eines kristallinen Feststoffes vom Schmelzpunkt 74-75°C.

Beispiel 2

Herstellung des Ausgangsproduktes der Formel

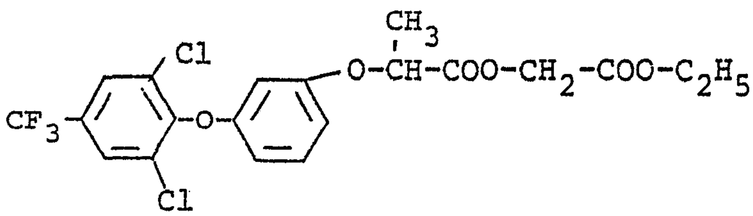

Zu einer Mischung aus 80,8 g (0,25 Mol) 3-(2,6-Dichlor-4-trifluormethyl-phenoxy)-phenol, 350 ml Aceton und 41,4 g (0,3 Mol) Kaliumcarbonat werden unter Rühren bei 50-60°C 63 g (0,2625 Mol) α-Brompropionsäure-(hydroxyessigsäure-ethylester)-ester getropft. Anschließend wird das Reaktionsgemisch weitere 7 Stunden bei 50-60°C gerührt. Dann wird aufgearbeitet, indem man das Reaktionsgemisch mit 500 ml Wasser und 500 ml Toluol versetzt, die Phasen trennt, die wäßrige Phase einmal mit 200 Toluol extrahiert, die vereinigten organischen Phasen einmal mit 200 ml 5 %iger wäßriger Natronlauge und einmal mit 200 ml Wasser wäscht und nach dem Trocknen über Natriumsulfat das Lösungsmittel unter vermindertem Druck abzieht. Man erhält auf diese Weise 90,8 g (75,5 % der Theorie) an 3-(2,6-Dichlor-4-trifluormethyl-phenoxy)-α-phenoxy-propionsäure-(hydroxyessigsäure-ethylester)-ester in Form eines gelben Öles.

Vergleichsbeispiele

Beispiel 3

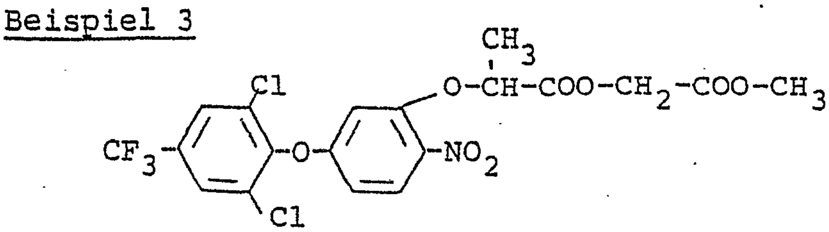

Zu einer Lösung von 4,7 g (0,01 Mol) 3-(2,6-Dichlor-4-trifluormethyl-phenoxy)-α-phenoxy-propionsäure-(hydroxyessigsäuremethylester)-ester in 50 ml 1,2-Dichlorethan wird bei einer Temperatur zwischen -10°C und -5°C unter Rühren eine Mischung aus 3,3 g (0,05 Mol) 96 %iger Salpetersäure und 2,6 g (0,025 Mol) 96 %iger Schwefelsäure getropft. Nach beendeter Zugabe wird weitere 3 Stunden bei -10°C bis -5°C gerührt. Anschließend versetzt man das Reaktionsgemisch unter Rühren und Kühlung mit 20 ml Wasser und trennt die Phasen. Die wäßrige Phase wird mit 25 ml 1,2-Dichlorethan extrahiert. Die vereinigten organischen Phasen werden einmal mit 25 ml gesättigter, wäßriger Natriumbicarbonat-Lösung und einmal mit 25 ml Wasser gewaschen und dann unter vermindertem Druck durch Abziehen des Lösungsmittels eingeengt. Es verbleiben 5,4 g eines rotbraunen, zähflüssigen Öles, das gemäß Gaschromatogramm zu 56,9 % aus 5-(2,6-Dichlor-4-trifluormethyl-phenoxy)-2-nitro-α-phenoxy-propionsäure-(hydroxyessigsäuremethylester)-ester besteht. Die Ausbeute beträgt danach 60,1 % der Theorie.

Beispiel 4

Herstellung der Verbindung der Formel

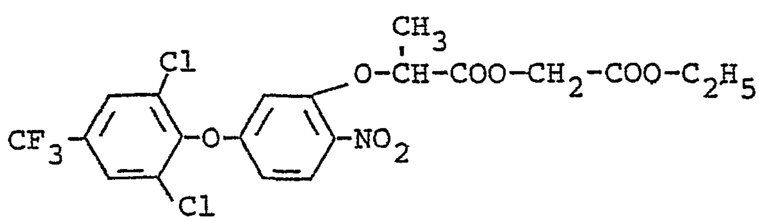

nach dem Verfahren gemäß DE-OS 29 06 087.

Eine Lösung von 15 g 5-(2,6-Dichlor-4-trifluormethyl-phenoxy)-2-nitro- α-phenoxy-propionsäurechlorid in 30 ml Toluol wird zu einer auf 0 bis 5°C gekühlten Lösung von 3,7 g Hydroxy-essigsäure-ethylester und 4 g Triethylamin in 70 ml Toluol tropfenweise gegeben. Das Reaktionsgemisch wird über Nacht bei Raumtemperatur gerührt, mit 300 ml Toluol verdünnt, neutral gewaschen, getrocknet, filtriert und eingeengt. Man erhält 13,5 g 5-(2,6-Dichlor-4-trifluormethyl-phenoxy)-2-nitro- α-phenoxy-propionsäure-(hydroxyessigsäureethylester)-ester zunächst in Form eines gelbstichigen Öles, das dann kristallisiert.
Schmelzpunkt: 75-76°C
Die Ausbeute an reiner Substanz errechnet sich zu 70,5 % der Theorie.

Patentansprüche

1. Verfahren zur Herstellung von 5-(2,6-Dichlor-4-trifluormethyl-phenoxy)-2-nitro- α-phenoxy-propionsäure-(hydroxyessigsäureethylester)-ester der Formel

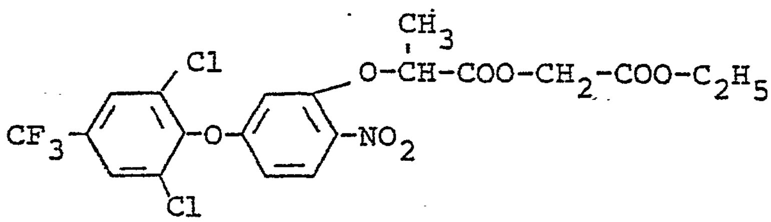

(I)

dadurch gekennzeichnet, daß man 3-(2,6-Dichlor-4-trifluormethyl-phenoxy)- α-phenoxy-propionsäure-(hydroxyessigsäureethylester)-ester der Formel

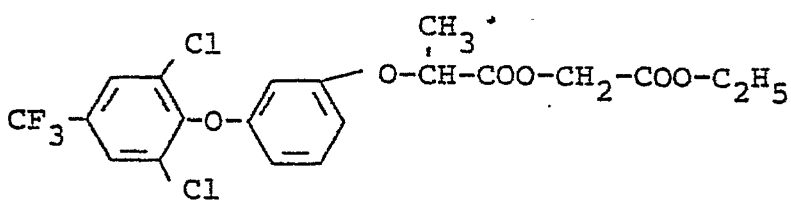

(II)

mit Salpetersäure in Gegenwart von Schwefelsäure sowie in Gegenwart von 1,2-Dichlorethan bei Temperaturen zwischen -20°C und +50°C umsetzt.

2. Verfahren gemäß Anspruch 1, dadurch gekennzeichnet, daß man Salpetersäure in Form von rauchender Salpetersäure einsetzt.

3. Verfahren gemäß Anspruch 1, dadurch gekennzeichnet, daß man Schwefelsäure in Form von konzentrierter oder zumindest stark konzentrierter Schwefelsäure einsetzt.

4. Verfahren gemäß Anspruch 1, dadurch gekennzeichnet, daß man die Umsetzung bei Temperaturen zwischen -15°C und +30°C durchführt.

5. Verfahren gemäß Anspruch 1, dadurch gekennzeichnet, daß man pro Mol an 3-(2,6-Dichlor-4-trifluormethyl-phenoxy)- α -phenoxy-propionsäure-(hydroxyessigsäureethylester)-ester der Formel (II) 1 bis 10 Mol Salpetersäure und 0,5 bis 10 Mol Schwefelsäure einsetzt.

Europäisches Patentamt

## EUROPÄISCHER RECHERCHENBERICHT

0111581
Nummer der Anmeldung

EP 82 11 1767

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
| A | EP-A-0 014 900 (BAYER AG)<br>* Beispiele 14 *<br>----- | 1-5 | C 07 C 76/02<br>C 07 C 79/46 |
| | | | RECHERCHIERTE SACHGEBIETE (Int. Cl. 3) |
| | | | C 07 C 76/02<br>C 07 C 79/46 |

Der vorliegende Recherchenbericht wurde für alle Patentansprüche erstellt.

| Recherchenort | Abschlußdatum der Recherche | Prüfer |
|---|---|---|
| BERLIN | 15-07-1983 | BREW C.H. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument